# EUROPEAN PATENT APPLICATION

(11) **EP 0 733 368 A1**
(43) Date of publication of application: **25.09.1996**
(21) Application number: 96301998.9
(22) Date of filing: 22.03.1996
(51) Int. Cl.: A61K 31/55, A61K 9/00, A61K 9/16

(54) **Granule fomulation for olanzapine**

(30) Priority: 24.03.1995 US 410265; 21.04.1995 US 426343
(71) Applicant: ELI LILLY AND COMPANY, Indianapolis, Indiana 46285 (US)
(72) Inventor: Lange, Hans Joerg, 20144 Hamburg (DE)
(74) Representative: Hudson, Christopher Mark

(57) **Abstract**

The invention provides a pharmaceutically elegant granule formulation of olanzapine and a process for providing a pharmaceutically acceptable liquid formulation of olanzapine. The solid granule formulation comprises olanzapine as an active ingredient, mannitol, hydroxypropylmethyl cellulose, and a pharmaceutically acceptable surfactant, provided that the size of the granules is such that not more than 5% are greater than 500 µm and not more than 10% are less than 75 µm.

## Description

This invention provides an improved pharmaceutically elegant granule formulation of 2-methyl-4-(4-methyl-1-piperazinyl)-10H-thieno[2,3-b][1,5]benzodiazepine, hereinafter referred to as olanzapine, and processes for the preparation thereof.

Olanzapine has shown great promise in the treatment of psychotic patients and is currently being evaluated for that purpose. Certain tablet formulations of olanzapine are known, as described in U.S. Patent No. 5,229,382. However, improved oral formulations were desired to offer a liquid dosage form providing an alternative to bolus presentation and a solution to compliance problems commonly encounted with psychotic patients.

Improved oral liquid formulations are desired in light of the moisture sensitive, metastable nature of olanzapine, due to the surprisingly potent nature of olanzapine, and the need to provide an acceptable shelf life for a liquid product. Such liquid formulations would prerably be prepared by dispersing an appropriate granule formulation in a liquid medium.

The granule formulation provided by the present invention addresses these needs. It is a low moisture content, free flowing granule formulation which facilitates uniform filling into sachets. The pharmaceutically elegant granules prepared using the formulation claimed herein are substantially sized between 75 and 500 µm, with not more than 5% greater than 500 µm and not more than 10% less than 75µm to provide ready dispersion in aqueous medium and a uniform reconstituted dosage. Additionally, the granule formulation of this invention is a dust free formulation which is stable in the dry form. Finally, the presently claimed granule formulation provides pharmaceutically acceptable stability when reconstituted.

The presently claimed invention provides a pharmaceutically elegant solid granule formulation for olanzapine comprising mannitol, hydroxypropyl methylcellulose, and a pharmaceutically acceptable surfactant.

The present invention provides a method for preparing a pharmaceutically acceptable liquid olanzapine formulation, comprising contacting a granule formulation of this invention with an aqueous medium.

Olanzapine, a potent compound showing great promise in the treatment of psychotic patients, has a tendency to undergo undesired polymorphic transformation, pharmaceutically undesired discoloration, and demands care to assure homogeneity of the finished solid formulation.

Applicants have discovered that olanzapine undergoes undesirable discoloration when contacted with certain excipients including powder blends. Further, the discoloration was exacerbated by ambient air conditions, at elevated temperatures, and by moist environments.

Although the discoloration phenomenon does not produce an increase in the number of total related substances, the browning and mottling appearance is not generally considered pharmaceutically acceptable for commercial purposes. Further, the discoloration is particularly disturbing when a tablet formulation is administered to a psychotic patient, which patient may be especially troubled by the changing appearance of their medication.

It is especially preferred that the formulation contain the most stable anhydrous form of olanzapine, referred to herein as Form II; however, other forms of olanzapine are contemplated. Form II has a typical x-ray powder diffraction pattern as represented by the following interplanar spacings:

| **d** |
|---|
| 10.2689 |
| 8.577 |
| 7.4721 |
| 7.125 |
| 6.1459 |
| 6.071 |
| 5.4849 |
| 5.2181 |
| 5.1251 |
| 4.9874 |
| 4.7665 |
| 4.7158 |
| 4.4787 |
| 4.3307 |
| 4.2294 |
| 4.141 |
| 3.9873 |

| **d** |
|---|
| 3.7206 |
| 3.5645 |
| 3.5366 |
| 3.3828 |
| 3.2516 |
| 3.134 |
| 3.0848 |
| 3.0638 |
| 3.0111 |
| 2.8739 |
| 2.8102 |
| 2.7217 |
| 2.6432 |
| 2.6007 |

A typical example of an x-ray diffraction pattern for Form II is as follows wherein d represents the interplanar spacing and I/I₁ represents the typical relative intensities:

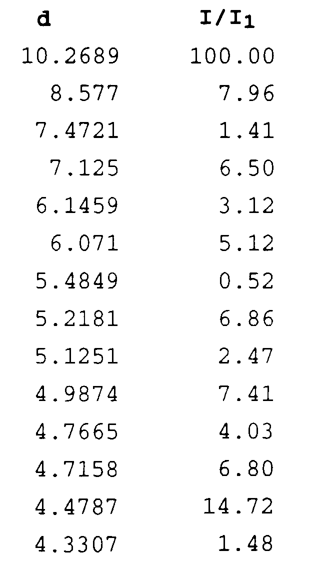

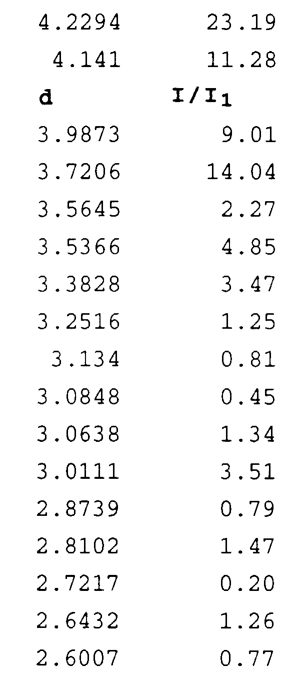

The x-ray diffraction patterns set out herein were obtained using a Siemens D5000 x-ray powder diffractometer having a copper K_{α} radiation source of wavelength, λ=1.541Å.

The formulation of the invention preferredly contains substantially pure Form II as the active ingredient.

As used herein "substantially pure" refers to Form II associated with less than about 5% undesired polymorphic form of olanzapine (herein referred to as "Undesired Form"), preferably less than about 2% Undesired Form, and more preferably less than about 1% Undesired Form. Further, "substantially pure" Form II will contain less than about 0.5% related substances, wherein "related substances" refers to undesired chemical impurities or residual solvent or water. In particular, "substantially pure" Form II preferably contain less than about 0.05% content of acetonitrile, more preferably, less than about 0.005% content of acetonitrile. Additionally, Form II preferredly contain less than 0.5% of associated water.

Form II is the most stable anhydrous form of olanzapine known and is therefore important for the commercial development of pharmaceutically elegant formulations. Olanzapine may form an undesired crystal form in the presence of certain solvents and excipients. Therefore, in making the compositions of the invention it is most desired to prepare the formulation using a method which does not require dissolution of the olanzapine substance. The desired Form II can be converted to less desirable polymorphic forms by contact with methylene chloride, for example. Additionally, for example, polyethylene glycol contact with the olanzapine substance produces undesired discoloration, particularly under moist conditions.

Olanzapine is effective over a wide dosage range, the actual dose administered being dependent on the condition being treated. For example, in the treatment of adult humans, dosages of from about 0.25 to 50 mg, preferably from 1 to 30 mg, and most preferably 1 to 20 mg per day may be used. A once a day dosage is normally sufficient, although divided doses may be administered. For treatment of central nervous system disorders, a dose range of from 1 to 30 mg, preferably 1 to 20 mg per day is suitable. Radiolabelled Form II 2-methyl-4-(4-methyl-1-piperazinyl)-10H-thieno-[2,3-b][1,5]benzodiazepine, can be detected in the saliva and thus the compound can potentially be monitored in patients to assess compliance.

In light of the potent nature of olanzapine, consistent dose uniformity is imperitive. In accordance with this invention, Applicants have discovered that mannitol, hydroxypropylmethyl cellulose, and an appropriate surfactant address the particular formulation needs associated with pharmaceutically elegant, stable, uniform olanzapine granules and provide a formulation which can be used to prepare a liquid dosage form.

A liquid dosage form can be particularly important for the treatment of psychotic patients who are commonly non-compliant. Psychotic patients often "cheek" a tablet formulation resulting in missed or eratic dosage of the therapeutic agent. The stable liquid formulation provided herein can provide greater compliance and ease of administration for the caregiver.

The present invention provides a formulation which enhances the stability of the pharmaceutically active agent and aids the speed of reconstitution. Applicants have discovered that mannitol is compatible with olanzapine; readily dissolves in water, has good taste properties and advantageous aqueous sorption characteristics.

Applicants discovered hydroxypropylmethyl cellulose could provide the fine granule size desired and does not adversely impact the physical characteristics of olanzapine.

Additionally, the claimed formulation must include an appropriate surfactant. It is essential that the surfactant is effective over a wide range of concentrations covering granule manufacture and reconstitution. Polysorbate 20 is a preferred surfactant and does not adversely impact the physcial characteristics of olanzapine.

A further object of this invention is to provide a granule formulation which is suitable for administration as a suspension or solution in an aqueous medium. It is particularly desired that the granule formulation will dissolve in fruit juice or other common acceptable liquid medium. The formulation claimed herein provides a pharmaceutically acceptable suspension when the granules are reconstituted in purified water. The granule formulation claimed herein completely dissolves in certain acidic fruit juices. For example, orange juice and grapefruit juice are two particularly preferred aqueous media. It is most preferred that the granule formulation of this invention will dissolve in other fruit juices such as apple, cranberry juice, strawberry juice, raspberry juice, grape juice, and other juices having a pH of about 6.5 or less.

The granules of this invention reconstitute within 60 seconds in 100ml water when gently stirred. A uniform dispersion is formed which eventually settles on standing. Gentle stirring resuspends the dispersion. Stability data of the dispersion indicates that the reconstituted liquid is acceptably stable for about 24 hours.

The present invention provides a method for preparing a pharmaceutically acceptable liquid olanzapine formulation, comprising contacting a granule formulation of this invention with an aqueous medium. Most preferably the aqueous medium is from about pH 4 to about pH 7.5. Especially preferred is pH 5 to about pH 7. When a solution is desired, about pH 6 to about pH6.5 is especially preferred. Such aqueous medium may be a fruit or vegetable juice having appropriate pH characteristics.

| **Ingredient** | **% by weight** |
|---|---|
| Olanzapine | about 0.5-about 2 |
| Mannitol | about 92-about 96 |
| hydroxypropyl methylcellulose | about 4-about6 |
| Polysorbate | about 0.009-about.05 |

A particularly preferred formulation is about 1% by weight olanzapine (w/w), about 93.99% (w/w) mannitol, about 5% (w/w) hydroxypropyl methylcellulose, and about 0.01% (w/w) polysorbate 20.

It was further preferred that the manufactured granule could be accurately and uniformly filled into sachets over the proposed range of product strengths.

A preferred formulation of the invention is a solid oral formulation providing from about 1 to about 20 mg or from about 1 to 10 mg of substantially pure Form II olanzapine per sachet as an effective amount of the active ingredient.

Most preferably, the solid granule formulation is contained in packaging materials which protect the formulation from moisture and light. For example, suitable packaging materials include polyester-foil/surlyn™ material sachets and other containers made of a material which inhibits the passage of light. The packaging may include a desiccant pack.

The materials for the present invention can be purchased or prepared by a variety of procedures well known to those of ordinary skill in the art. The olanzapine compound can be prepared as described by Chakrabarti in U.S. Patent No 5,229,382 ('382), herein incorporated by reference in its entirety.

Compound characterization methods include, for example, x-ray powder pattern analysis, thermogravimetric analysis (TGA), differential scanning calorimetery (DSC), titrametric analysis for water, and H¹-NMR analysis for solvent content.

Solid olanzapine formulations were studied to assure that the Form II polymorph was substantially pure using ¹³C Cross polarization / magic angle spinning (CP/MAS) NMR. Spectra are obtained using a Varian Unity 400 MHz spectrometer operating at a carbon frequency of 100.577 MHz and equipped with a complete solids accessory and Varian 5 mm or 7 mm VT CP/MAS probes. Measurement conditions are optimized for Olanzapine Form II and are as follows: 90° proton r.f. pulse 4.5 ms, contact time 1.1 ms, pulse repetition time 5 s, MAS frequency 7.0 kHz, spectral width 50 kHz, and acquisition time 50 ms. Chemical shifts are referenced to the CH₃ of hexamethylbenzene (d = 17.3 ppm) by sample replacement.

The formulations of this invention provide substantially pure Form II polymorph in a pharmaceutically elegant formulation without producing undesired polymorphic transformation.

The following examples are provided for purposes of illustration and are not to be construed as limiting the scope of the claimed invention.

### Preparation 1

### Technical Grade olanzapine

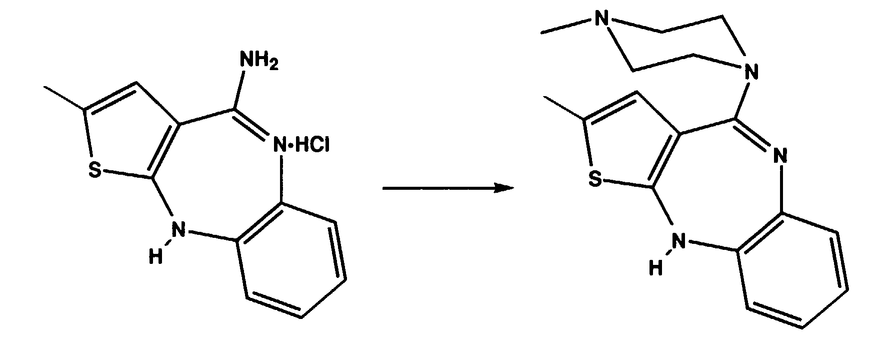

### Intermediate 1

In a suitable three neck flask the following was added:

| | |
|---|---|
| Dimethylsulfoxide (analytical) : | 6 volumes |
| Intermediate 1 : | 75 g |
| N-Methylpiperazine (reagent) : | 6 equivalents |

Intermediate 1 can be prepared using methods known to the skilled artisan. For example, the preparation of the Intermediate 1 is taught in the '382 patent.

A sub-surface nitrogen sparge line was added to remove the ammonia formed during the reaction. The reaction was heated to 120°C and maintained at that temperature throughout the duration of the reaction. The reactions were followed by HPLC until ≤ 5% of the intermediate 1 was left unreacted. After the reaction was complete, the mixture was allowed to cool slowly to 20°C (about 2 hours). The reaction mixture was then transferred to an appropriate three neck round bottom flask and water bath. To this solution with agitation was added 10 volumes reagent grade methanol and the reaction was stirred at 20°C for 30 minutes. Three volumes of water was added slowly over about 30 minutes. The reaction slurry was cooled to zero to 5°C and stirred for 30 minutes. The product was filtered and the wet cake was washed with chilled methanol. The wet cake was dried in vacuo at 45°C overnight. The product was identified as technical olanzapine.
Yield: 76.7%; Potency: 98.1%

### Preparation 2

### Form II

A 270 g sample of technical grade 2-methyl-4-(4-methyl-1-piperazinyl)-10H-thieno[2,3-b][1,5]benzodiazepine was suspended in anhydrous ethyl acetate (2.7 L) . The mixture was heated to 76°C and maintained at 76°C for 30 minutes. The mixture was allowed to cool to 25°C. The resulting product was isolated using vacuum filtration. The product was identified as Form II using x-ray powder analysis.
Yield: 197 g.

The process described above for preparing Form II provides a pharmaceutically elegant product having potency ≥ 97%, total related substances < 0.5% and an isolated yield of > 73%.

### EXAMPLE 1

### Olanzapine Granules

The granules were produced by blending the mannitol and Hydroxymethyl propyl cellulose in a high shear mixer; granulating with the aqueous suspension of olanzapine and polysorbate 20; wet sized and subsequently dried in a fluid bed dryer. These are dry sized and reblended prior to packaging.
**1a. 250mg total wt. Sachets**

| **INGREDIENT** | **MG/SACHET** |
|---|---|
| **Active** | |
| Olanzapine | 2.50 |
| | |
| **Other Ingredients** | |
| Mannitol | 234.97 |
| Hydroxypropyl methyl cellulose 3cps | 12.50 |
| Polysorbate 20 | 0.028 |

**1b. 750mg total wt.Sachets**

| **INGREDIENT** | **MG/SACHET** |
|---|---|
| **Active** | |
| Olanzapine | 7.50 |
| | |
| **Other Ingredients** | |
| Mannitol | 704.93 |
| Hydroxypropyl methyl cellulose 3cps | 37.49 |
| Polysorbate 20 | 0.08 |

**1c. 1000mg total wt.Sachets**

| **INGREDIENT** | **MG/SACHET** |
|---|---|
| **Active** | |
| Olanzapine | 10.00 |
| | |
| **Other Ingredients** | |
| Mannitol | 939.90 |
| Hydroxypropyl methyl cellulose 3cps | 49.99 |
| Polysorbate 20 | 0.11 |

Such granules are most preferably contacted with an acidic medium if a suspension or solution is desired.

### EXAMPLE 2

### Olanzapine Granules

The granules were produced by blending the mannitol and Hydroxymethyl propyl cellulose in a fluidized bed drier; granulating with the aqueous suspension of olanzapine and polysorbate; wet sized and subsequently dried in a fluid bed dryer. These are dry sized and reblended prior to packaging.
**1a. 250mg total wt. Sachets**

| **INGREDIENT** | **MG/SACHET** |
|---|---|
| **Active** | |
| Olanzapine | 2.50 |
| | |
| **Other Ingredients** | |
| Mannitol | 234.97 |
| Hydroxypropyl methyl cellulose 3cps | 12.50 |
| Polysorbate 20 | 0.028 |

**1b. 750mg total wt.Sachets**

| **INGREDIENT** | **MG/SACHET** |
|---|---|
| **Active** | |
| Olanzapine | 7.50 |
| | |
| **Other Ingredients** | |
| Mannitol | 704.93 |
| Hydroxypropyl methyl cellulose 3cps | 37.49 |
| Polysorbate 20 | 0.08 |

**1c. 1000mg total wt.Sachets**

| **INGREDIENT** | **MG/SACHET** |
|---|---|
| **Active** | |
| Olanzapine | 10.0 |
| | |
| **Other Ingredients** | |
| Mannitol | 939.90 |
| Hydroxypropyl methyl cellulose 3cps | 49.99 |
| Polysorbate 20 | 0.11 |

Such granules are most preferably contacted with an acidic medium if a solution is desired.

## Claims

1. A solid granule formulation comprising olanzapine as an active ingredient, mannitol, hydroxypropylmethyl cellulose, and a pharmaceutically acceptable surfactant, provided that the size of the granules is such that not more than 5% are greater than 500 µm and not more than 10% are less than 75µm.

2. A granule formulation of **Claim 1** wherein said surfactant is polysorbate.

3. A granule formulation of **Claim 2** wherein said formulation comprises
| **Ingredient** | **% by weight** |
|---|---|
| Olanzapine | about 0.5-about 2 |
| Mannitol | about 92-about 96 |
| hydroxypropyl methylcellulose | about 4-about 6 |
| Polysorbate | about 0.009-about.05 |

4. A granule formulation of **Claim 3** wherein said formulation comprises about 1% by weight olanzapine (w/w), about 93.99% (w/w) mannitol, about 5% (w/w) hydroxypropyl methylcellulose, and about 0.01% (w/w) polysorbate 20.

5. A granule formulation as claimed in any one of **Claims 1** to **4** wherein the active ingredient is substantially pure Form II olanzapine polymorph having a typical x-ray powder diffraction pattern as represented by the following interplanar spacings:

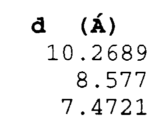

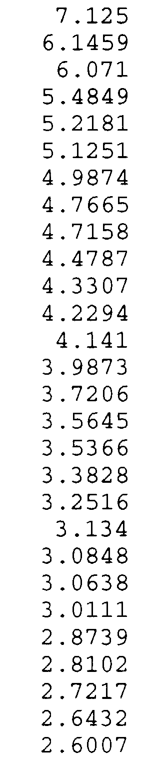

6. A process for preparing a pharmaceutically acceptable liquid formulation of olanzapine comprising contacting a granule formulation as claimed in any one of **Claims 1** to **5** with an aqueous medium of about pH 4 to about pH 7.5.

7. A process as claimed in **Claim 6** wherein said aqueous medium is acidic mineral water having a pH about 6 to about 6.5.

8. A process of **Claim 6** wherein said aqueous medium is a fruit or vegetable juice.

9. A process for preparing a solid granule formulation having an effective amount of olanzapine as an active ingredient, mannitol, hydroxypropylmethyl cellulose, and a pharmaceutically acceptable surfactant, provided that the size of the granules is such that not more than 5% are greater than 500 µm and not more than 10% less are than 75µm comprising wet sizing the mixture and drying the sized granules.

10. A solid granule formulation having an effective amount of olanzapine as an active ingredient, mannitol, hydroxypropylmethyl cellulose, and a pharmaceutically acceptable surfactant, provided that the size of the granules is such that not more than 5% are greater than 500 µm and not more than 10% less are than 75 µm for use in treating a condition selected from the group consisting of psychosis, schizophrenia, a schizophriform disorder, mild anxiety, a gastrointestinal disorder, and acute mania.
